## Europäisches Patentamt
## European Patent Office
## Office européen des brevets

(19)

(11) Veröffentlichungsnummer : **0 394 428 B1**

(12)

# EUROPÄISCHE PATENTSCHRIFT

(45) Veröffentlichungstag der Patentschrift :
**22.02.95 Patentblatt 95/08**

(51) Int. Cl.⁶ : **G01S 7/52**, G01S 15/89,
// A61B8/06

(21) Anmeldenummer : **89912415.0**

(22) Anmeldetag : **19.10.89**

(86) Internationale Anmeldenummer :
**PCT/EP89/01246**

(87) Internationale Veröffentlichungsnummer :
**WO 90/04793 03.05.90 Gazette 90/10**

(54) **VERFAHREN UND VORRICHTUNG ZUM MESSEN VON ZWEIDIMENSIONALEN REFLEKTIERENDEN STRUKTUREN.**

Teilanmeldung 94109331.2 eingereicht am 19/10/89.

(30) Priorität : **19.10.88 CH 3886/88**

(43) Veröffentlichungstag der Anmeldung :
**31.10.90 Patentblatt 90/44**

(45) Bekanntmachung des Hinweises auf die Patenterteilung :
**22.02.95 Patentblatt 95/08**

(84) Benannte Vertragsstaaten :
**AT BE CH DE FR GB IT LI LU NL SE**

(56) Entgegenhaltungen :
**DE-A- 3 605 163
US-A- 4 596 145
US-A- 4 608 868
US-A- 4 800 891**

(56) Entgegenhaltungen :
**Ultrasonic Imaging,vol.8,No.2,April 1986 Academic Press(Duluth,MN,US) O.Bonnefous et al.: "Time domain formulation of pulse-Doppler ultrasound and blood velocity estimation by cross correlation",pages 73-85 see the whole article**

(73) Patentinhaber : **Institut für Biomedizinische Technik und Medizinische Informatik der Universität und ETH Zürich
Moussonstrasse 18
CH-8044 Zürich (CH)**

(72) Erfinder : **MOSER, Urs
Vogelsangstrasse 46
CH-46 8006 Zürich (CH)**

(74) Vertreter : **Troesch Scheidegger Werner AG Patentanwälte,
Siewerdtstrasse 95,
Postfach
CH-8050 Zürich (CH)**

## Beschreibung

Die vorliegende Erfindung betrifft ein Verfahren und eine Messanordnung nach dem Oberbegriff von Anspruch 1 bzw. Anspruch 14.

Insbesondere im Zusammenhang mit der Ultraschall-Puls-Dopplertechnik zur Messung von Strömungsgeschwindigkeiten in einem Streuzentren mitführenden Fluidum ist es seit über zwei Jahrzehnten bekannt (Baker, "Pulsed Ultrasonic Blood Flow Sensing", IEEE Trans. SU-17/3, p. 170 (1970)), durch Auswerten der Doppler-Frequenz an einem aus einer bestimmten Distanz stammenden Echosignal bzw. dessen Phasenlage bezüglich eines stabilen Referenzsignals, die Geschwindigkeit des Fluidums zu bestimmen. Dabei wird aufgrund des Dopplereffektes nur die in Strahlrichtung zeigende Geschwindigkeitskomponente ermittelt.

Aufgrund der an einer zweidimensionalen Schicht bezüglich eines Empfängers bestehenden unterschiedlichen Distanzen konnte, nach der im obgenannten Artikel bekannten Technik, eine zweidimensionale Struktur nicht gemessen werden, auch bei Anwendung des erwähnten, bekannten Verfahrens auf eine stationäre.

In einem weiter entwickelten, sog. vielkanaligen Gerät (McLeod, "A Multiple Gate Pulsed Doppler Flowmeter", Proc. IEEE Ultrasonic Symp. Miami, (1971)) wurden, sequentiell, die Echos ausschliesslich aus verschiedenen Distanzen ausgewertet und quasi simultan die Resultate angezeigt, entsprechend einem Geschwindigkeitsprofil entlang des Beschallungsstrahls. Auch bei diesem Verfahren ist aufgrund obgenannter Distanzverhältnisse an einer zweidimensionalen Schicht und des eigentlich eindimensionalen Messprinzips, eine Messung eingangs genannter Art nicht möglich.

Aus der DE-PS 24 06 630 wurde im weiteren ein Verfahren zur Messung eines Geschwindigkeitsprofils eines reflektierende Strukturen mitführenden, fliessenden Fluides, aus der Auswertung von Schall- oder Ultraschallechos bekannt, durch welches es gelang, das Geschwindigkeitsprofil ohne eigentliche Distanzvorgabe zu ermitteln. Auch mit diesem Verfahren ist es aber nicht möglich, aufgrund obgenannter Distanzverhältnisse eine zweidimensional ausgedehnte Schicht bezüglich reflektierender Strukturen auszumessen, im Falle, dass das genannte Vorgehen auf stationäre Strukturen angewandt wird.

Im weiteren ist es, z.B. aus der DE-OS 3 605 163, bekannt, mittels sog. Phased-Array-Sektorscannern, den Schallstrahl in einer die Sender/Ziel-Achse enthaltenen Ebene, durch unterschiedliche Senderanregung, unterschiedlich auszulenken und so Streuzentrengeschwindigkeiten jeweils entlang der momentan angesteuerten Beschallungsachse, sequentiell in verschiedenen Richtungen in besagter Ebene zu ermitteln, so dass sequentiell in dieser Schnittebene, in dem dem Fachmann bekannten "B-Modus" das zweidimensionale Geschwindigkeitsfeld abgetastet wird.

Bei diesem Vorgehen ist, auch bei stationären Strukturen, eine Echografie nur einer Schnittfläche, welche in der genannten Ablenkebene liegt, möglich. Bei Anwendung des genannten Verfahrens für seinen eigentlichen Zweck, nämlich die Messung eines Geschwindigkeitsprofils, entsteht ein weiteres Problem: Für eine Echtzeitmessung muss die Messzeit für das Abtasten in der Ebene limitiert werden, so dass entweder pro momentane Strahlrichtung nur kurze Messzeitspannen zur Verfügung stehen oder aber, bei Verlängerung der genannten Zeitspannen, nur einige wenige Abtastrichtungen ausgemessen werden können.

Bei den sog. Color-Doppler-Geräten (Chihiro, "Real-Time Two-Dimensional Blood Flow Imaging Using an Autocorrelation Technique", IEEE Trans. on Sonics and Ultrasonics, Vol, SU 32, No. 3, (1985)) wurde vor allem die Messzeit in eine jeweilige Strahlrichtung stark limitiert, so dass sich, aufgrund der Unschärferelation, die Dopplerfrequenz nur sehr ungenau bestimmen lässt.

In "Ultrasonic Imaging", Bd. 8, Nr. 2, April 1986, p. 79 ist beispielsweise beschrieben, mittels welcher mathematischer Verknüpfungen aus sequentiell anfallenden, störunterdrückten Echosignalen auf die Blutströmungsgeschwindigkeit in einem beschallten Volumenelement geschlossen werden kann.

Schliesslich ist aus der US-A 4 596 145 ein Verfahren bzw. eine Messanordnung der eingangs genannten Art bekannt.

Dabei werden Empfangssensoren einer zweidimensionalen, ebenen Anordnung in Gruppen unterteilt und die Sensoren jeder Gruppe durch Vorgabe Laufzeitunterschiede-berücksichtigender Verzögerungszeiten auf ein Volumenelement elektronisch fokussiert, bei allen Gruppen zugeordneten, gleichen Verzögerungszeiten, alle Gruppen auf in einer Ebene parallel zur ebenen Anordnung liegende Volumenelemente. Wegen der Sensor-Gruppierung ist dabei die Menge gleichzeitiger erfasster Volumenelemente bei gegebener Sensorzahl beschränkt und zudem ergibt sich bei der elektronischen Fokussierung deshalb ein relativ schlechtes SNR, weil die Fokussierung nur mittels der relativ geringen Gruppenmitgliederzahl an Sensoren erfolgt.

Die vorliegende Erfindung setzt sich zum Ziel, ein Verfahren eingangs genannter Art bzw. eine entsprechende Vorrichtung zu schaffen, mittels welchen, eingesetzt auch auf stationäre Strukturen, deren strukturiertes Echo in Echtzeit und unter Vermeidung der obgenannten Nachteile erfasst werden kann, bzw. bei Einsetzen des genannten Verfahrens bzw. der entsprechenden Anordnung auf bewegte Strukturen, deren zweidimensionales Geschwindigkeitsfeld in Quasi-Echtzeit und unter Vermeidung der obgenannten Nachteile erfasst werden kann.

Bezüglich der erwähnten stationären Strukturen eingesetzt, wird diese Aufgabe durch das Verfahren nach dem Wortlaut des kennzeichnenden Teils von Anspruch 1 gelöst bzw. durch die Anordnung gemäss demjenigen von Anspruch 14. Eine bevorzugte Weiterbildung zum Messen eines zweidimensionalen Geschwindigkeitsfeldes zeichnet sich verfahrensmässig nach dem Wortlaut von Anspruch 2 bzw. anlagemässig gemäss Anspruch 15 aus.

Grundsätzlich werden mithin, sei dies in der Echografie-Anwendung, auch für stationäre Strukturen, oder zur Geschwindigkeitsmessung, die erwähnten Nachteile dadurch behoben, dass gleichzeitig das ganze interessierende, zweidimensionale Gebiet beschallt wird und, abgesehen von Laufzeitdifferenzen, welche durch unterschiedliche räumliche Lagen von Volumenelementen in der interessierenden Schicht relativ zu Sender und/oder Empfänger bedingt sind, eine bis auf Rechenzeiten simultane Auswertung der Echos aus dem gesamten interessierenden Zielgebiet über die ganze Empfangsanordnung vorgenommen wird.

Obwohl es durchaus möglich ist, das Sende-Wellenpaket so zu erzeugen, dass es die Schicht nicht kohärent erreicht, um darnach diese Nicht-Kohärenz (Phasenungleichheit) rechnerisch, durch Berücksichtigung von lokal unterschiedlichen Distanzen zwischen Sender und Volumenelementen der Schicht, zu berücksichtigen, wird gemäss Wortlaut von Anspruch 3 in einer bevorzugten Ausführungsvariante das Sende-Wellenpaket so erzeugt, dass es die Schicht mindestens genähert kohärent (phasengleich) erreicht, womit die Berücksichtigung der genannten unterschiedlichen Sende/Volumenelement-Distanzen entfällt.

Dabei wird das Sende-Wellenpaket bevorzugterweise nach dem Wortlaut von Anspruch 4 erzeugt, womit es durch die zeit- und phasenrichtige Anregung der vorgesehenen Mehrzahl von Quellen möglich wird, gegebenenfalls vorliegende Unterschiede der Distanzen zwischen einzelnen Quellen und einzelnen Volumenelementen der Schicht zu berücksichtigen. Durch das zeit- und phasenrichtige Anregen wird erreicht, dass, wie auch die interessierende Schicht räumlich bezüglich der Mehrzahl vorgesehener Quellen liegen mag, das durch die Mehrzahl von Quellen gesamthaft erzeugte Sende-Wellenpaket, wie bevorzugterweise gefordert, kohärent an der interessierenden Schicht auftrifft.

Einfachere Verhältnisse entstehen, wenn nach dem Wortlaut von Anspruch 5 vorgegangen wird.

Obwohl die Mehrzahl vorgesehener Sensoren durchaus auch in einer dreidimensionalen Anordnung, räumlich, angeordnet werden kann und die aufgrund der Zielschicht und der räumlichen Sensoranordnung entsprechend auftretenden Distanzunterschiede rechnerisch berücksichtigt werden können, wird in einer bevorzugten Ausführungsvariante vorgeschlagen, gemäss Wortlaut von Anspruch 6 vorzugehen.

Da, mit einer im wesentlichen ebenen Senderanordnung, wie auch mit einer im wesentlichen ebenen Sensorenanordnung, trotzdem beliebig im Raum liegende Schichten durch rechnerische Berücksichtigung der entsprechenden Sender-Volumenelemente bzw. Volumenelement-Sensoren-Distanzverhältnisse möglich ist, ergibt sich nach dem Wortlaut von Anspruch 7 ein bevorzugtes Vorgehen dadurch, dass Senden und Empfangen von derselben Anordnung aus bewerkstelligt wird und hier eine räumliche, vorzugsweise ebene Anordnung von Sende- und Empfangs-Transducern vorgesehen wird. Damit ergibt sich auch ein kompakter Aufbau von Anregungs- und Auswerte-Elektronik.

Das Sende-Wellenpaket wird, sofern es mittels einer Mehrzahl von Quellen erzeugt wird, bevorzugterweise nach dem Wortlaut von Anspruch 8 erzeugt. Darnach werden gegebenenfalls notwendige Zeit- und Phasenverschiebungen der Anregungen der einzelnen Quellen mit Bezug auf das erwähnte Referenzsignal definiert.

Im weiteren wird das Referenzsignal dem Wortlaut von Anspruch 9 folgend, zur Demodulation der Echosignale eingesetzt, wobei vorzugsweise diese Demodulation auf der elektrischen Seite des eigentlich als Schalldruck/elektrischer Wandler ausgebildeten Sensors vorgenommen wird.

Im weiteren werden gemäss Anspruch 10 die Echos, insbesondere die Volumenelementechos, von jedem Sensor digitalisiert und gespeichert, und zwar bevorzugterweise nach der Demodulation der Echosignale.

Durch das weitere bevorzugte Vorgehen gemäss Wortlaut von Anspruch 11 wird erreicht, dass Echos von einem Volumenelement, die auf alle Sensoren auftreffen, rechnerisch gleichphasig gemacht werden, womit eine einfache Möglichkeit geschaffen wird, je alle Echos von je einem Volumenelement über alle Sensoren gemeinsam auszuwerten.

Grundsätzlich werden gemäss Anspruch 12, durch Bekanntsein der entsprechenden Echo-Empfangszeiten bzw. Distanzen an jedem Sensor, alle Volumenelementechos an jedem Sensor identifiziert und darnach so miteinander verrechnet, dass die an allen Sensoren auftreffenden Echos, jeweils von einem Volumenelement, verstärkt werden, gegenüber den von anderen Volumenelementen an den Sensoren auftreffenden Echos.

Dies wird für alle Volumenelemente durchgeführt, womit letztendlich für jedes Volumenelement von der gesamten Sensoranordnung das Gesamt-Volumenelementechosignal zur Verfügung steht.

Wird, wie oben erläutert wurde, rechnerisch Kohärenz der Echos von einem Volumenelement an allen Sensoren erreicht, so wird die verstärkende, "signal to noise"-verbessernde Verrechnung, vorzugsweise nach dem Wortlaut von Anspruch 13, realisiert.

Diese sensorspezifischen Echos, woraus die Volumenelementechos identifiziert werden, dienen, gespeichert, als Datenbasis für alle Identifikations- und Verrechnungsschritte zum Erhalt der der Anzahl Volumenelemente entsprechenden Anzahl Gesamt-Volumenelementechos, damit des Schichtechos.

Zum obgenannten Messen des zweidimensionalen Geschwindigkeitsfeldes der Struktur in der erwähnten Schicht wird, wie erwähnt, gemäss Wortlaut von Anspruch 2 vorgegangen, indem nun mindestens zwei Sende-Wellenpakete in erläuterter Art und Weise erzeugt werden, zeitgestaffelt, und ihre Echos je nach den bisherigen Erläuterungen ausgewertet werden. Durch zusätzliches Auswerten der Unterschiede, mindestens zweier Sätze von Gesamtvolumenelementechos, wird die Geschwindigkeit der Strukturen in den jeweiligen Volumenelementen ermittelt und, über alle Volumenelemente, schliesslich die Geschwindigkeitsverteilung bzw. das Geschwindigkeitsfeld der Struktur über der Schicht.

Die mit dem erfindungsgemässen Verfahren, sei dies gerichtet auf stationäre Strukturen, im Sinne der Echografie oder und vor allem, gerichtet auf bewegte, zur Erfassung des Geschwindigkeitsfeldes erwirkten Vorteile, bestehen insbesondere darin, dass, für Echtzeitmessungen, das Problem der knappen Messzeit eliminiert wird, indem das ganze interessierende, in die genannten Abtastvolumina aufgeteilte Gebiet simultan erfasst wird. Durch die markant verkürzte Messzeit ist es auch im Echtzeitbetrieb möglich, sowohl eine gute örtliche Auflösung, wie auch eine hohe Genauigkeit, insbesondere auch bei der Geschwindigkeitsmessung, zu erreichen.

Ein weiterer Vorteil des erfindungsgemässen Verfahrens besteht darin, dass, im Gegensatz zum oben erwähnten Color-Doppler-Verfahren, die abgetastete Schicht nicht die Hauptbeschallungsachse enthält bzw. enthalten muss, sondern zu dieser z.B. senkrecht, in wählbarem Abstand von der Sensoranordnung, stehen kann. Dabei ist darauf hinzuweisen, dass durch Vorgabe der Distanzen jedes Sensors von jedem Volumenelement die zweidimensionale Messung unabhängig von der Lage der Schicht zur Hauptbeschallungsachse der Senderanordnung bzw. der Transduceranordnung wird. Mit Hilfe der Distanzvorgabe wird eigentlich festgelegt, welche Schicht ausgemessen werden soll bzw. wie sie im Raume bezüglich der Senderanordnung liegen soll. Dabei versteht es sich von selbst, dass durch Wechseln solcher "Distanzen-Tabellen" eine Schicht-für Schicht-Messung in rascher Abfolge möglich ist.

Der Volumenstrom kann durch Summation der Volumenelement-Geschwindigkeiten bestimmt werden. Hierzu sei auf Moser, "Inhärente Grenzen von Ultraschall-Blutflussmessverfahren", Dissertation, ETH No. 8567, 1988, hingewiesen.

Die Erfindung wird anschliessend beispielsweise anhand von Figuren erläutert.

Es zeigen:

Fig. 1    schematisch die räumliche Anordnung einer bevorzugten Sender/Empfängeranordnung in Form einer Transducermatrix sowie einer auszumessenden Schicht, im Spezialfall eine zur Transducerebene parallele Schicht,

Fig. 2    eine schematische Darstellung der Anregungseinheiten sowie der Echosignal-Auswerteeinheit, zur Signaldigitalisierung und zum Weitermultiplexen der digitalisierten Signale,

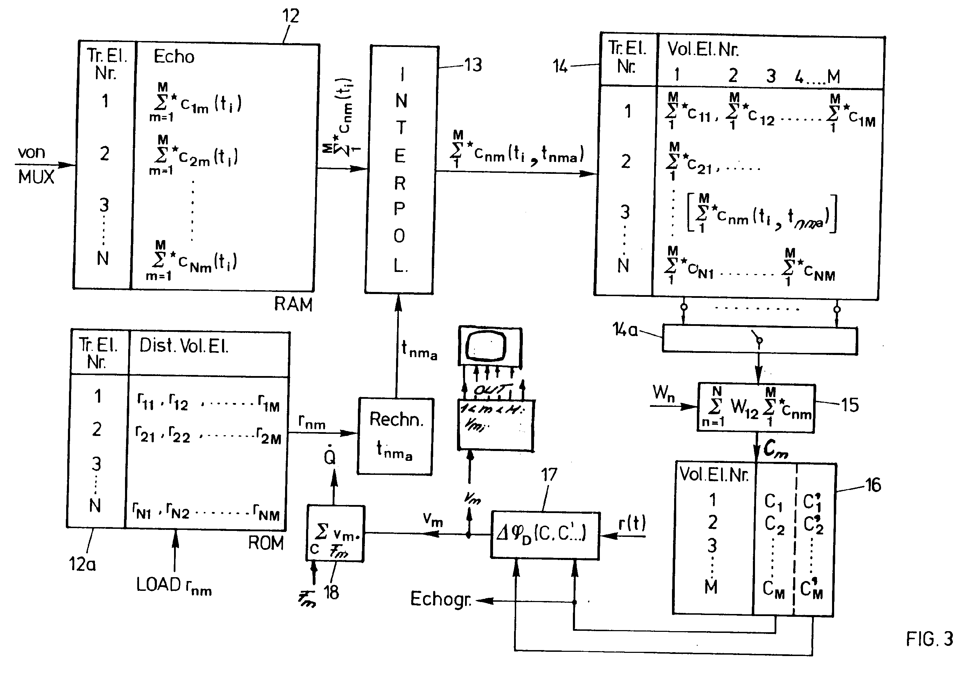

Fig. 3    in Fortsetzung von Fig. 2 die Weiterverarbeitung der Signale ausgangsseitig des Multiplexers gemäss Fig. 2, anhand eines Signalfluss-Funktionsblock-Diagrammes,

Fig. 4a    die Enveloppen-Funktion erfindungsgemäss erzeugter Sende-Wellenpakete über der Zeitachse,

Fig. 4b    das Wellen- bzw. Trägersignal,

Fig. 4c    das mit der Enveloppen-Funktion amplitudenmodulierte Trägersignal gemäss Fig. 4b zur Erzeugung der Sende-Wellenpakete,

Fig. 4d    wiederum über der Zeitachse, ein am Empfangssensorelement von einem Volumenelement aus der Ziel-Schicht erhaltenes Wellenpaket,

Fig. 4e    das Enveloppen-Signal des Echowellenpakets gemäss Fig. 4d, und diesbezüglich die Abtastzeiten $t_i$ gemäss Fig. 2.

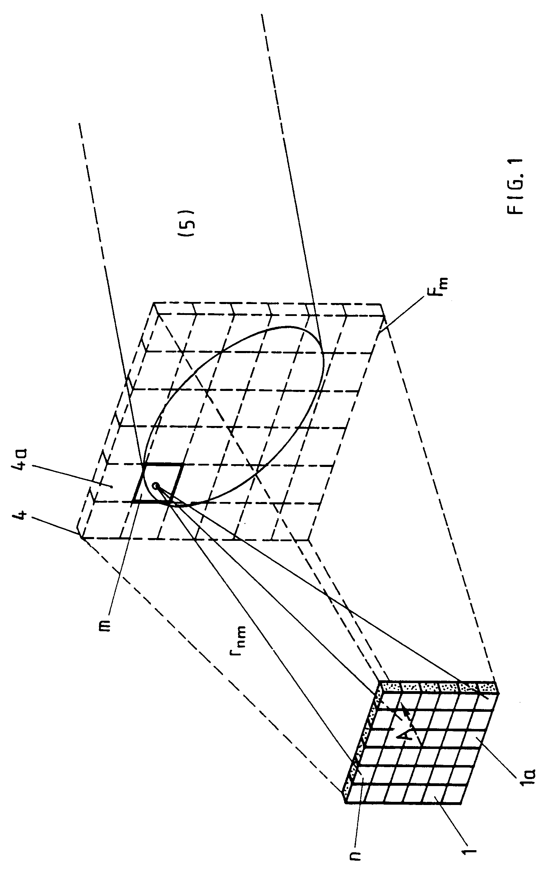

In Fig. 1 ist, schematisch, eine bevorzugte, erfindungsgemässe Anordnung von Quellen zur Erzeugung des Sende-Wellenpakets und von Sensoren zum Empfang der rückgestreuten Echos dargestellt. Bei diesem bevorzugten Ausführungsbeispiel dienen als Sender-Quellen und als Empfangssensoren die gleichen Elemente, sog. Transducer 1, welche in der bevorzugten Ausführungsform im wesentlichen in einer Ebene, matrixförmig, 1a, angeordnet sind.

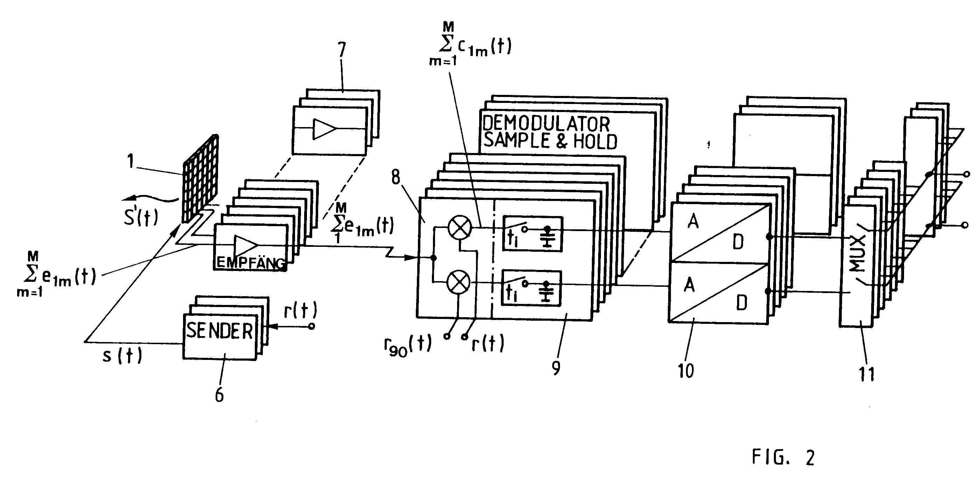

Wie aus Fig. 2 ersichtlich, werden die Transducerelemente 1 mittels eines oder mehrerer Sender 6 zur gemeinsamen Abgabe eines Sende-Wellenpaketes s'(t) angeregt. Wie wiederum aus Fig. 2 ersichtlich, ist den Transducerelementen 1 gemäss Fig. 1 je ein Empfänger 7 zugeschaltet, welcher die vom Transducerelement 1 in elektrische Signale gewandelten Echosignale verstärkt.

Die Ausgangssignale der Empfänger 7 werden, wie in vielkanaligen Doppler-Geräten üblich (siehe "McLeod"), mit Hilfe eines Synchrondemodulators orthogonal ins Basisband gemischt. Hierzu werden zwei orthogonale Referenzsignale r(t) und $r_{90}$(t) dem Synchrondemodulator 8 zugeführt, wobei das Referenzsignal r(t) auch zur Anregung der Transducerelemente 1 über die Sender 6 eingesetzt wird.

Zu wenigen, z.B. vier, vorgegebenen Zeitpunkten $t_i$ werden die am Synchrondemodulator 8 demodulierten orthogonalen Echosignale von jedem Transducerelement 1 an einem Abtast- und Haltekreis 9 abgetastet und je einem A/D-Wandler 10 zugeführt, dort digitalisiert. Die an den A/D-Wandlern 10 digitalisierten Werte gelangen über einen Multiplexer 11 zur Zwischenspeicherung in einen Speicher 12, bevorzugterweise einen RAM-Speicher.

Die Transducerelemente 1 erzeugen ein Wellenpaket s'(t) - mindestens eines bei Einsatz für Echografie, mindestens zwei zur Geschwindigkeitsfelderfassung. Mit der Trägerfrequenz $\omega_o$ kann das Sende-Wellenpaket wie folgt angeschrieben werden:

$$s'(t) = A(t)e^{j\omega_o t} \quad (1)$$

Dabei bezeichnen:

A(t): die Enveloppe des Sende-Wellenpakets

t : die Zeit

$\omega_o$ : die Trägerkreisfrequenz

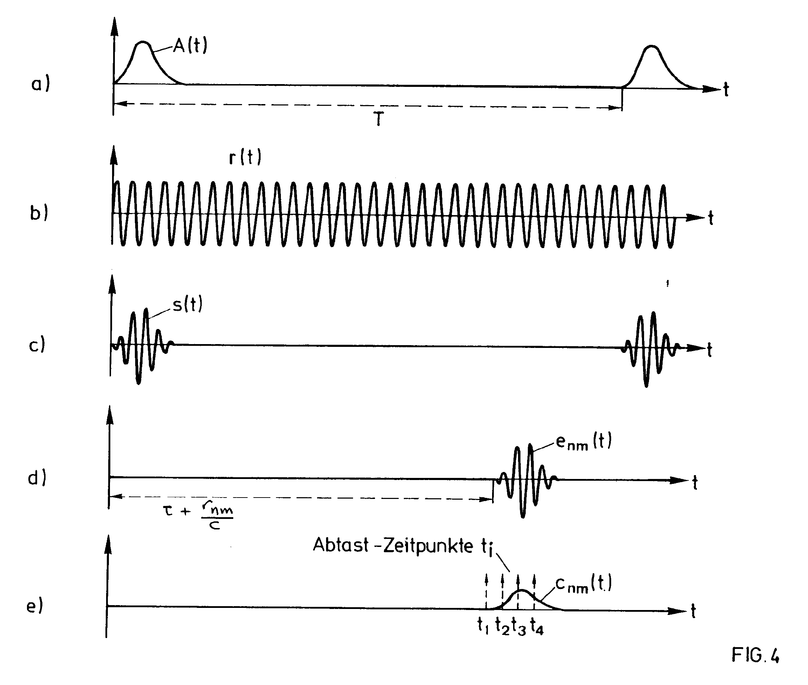

Die Enveloppe A(t) des Sende-Wellenpaketes gemäss Fig. 4a ist, verglichen mit der Repetitionszeit T bei Geschwindigkeitsfeldmessung, nur kurzzeitig verschieden von Null. Das Sende-Wellenpaket s'(t) kann als Produkt der Enveloppe A(t) mit dem quarzstabilen Referenzsignal r(t) angesehen werden, wobei das Referenzsignal r(t) in Fig. 4b, das resultierende Sende-Wellenpaket in Fig. 4c dargestellt sind.

Das Referenzsignal r(t) kann dargestellt werden:

$$r(t) = e^{j\omega_o t} \quad (2)$$

Das Anregungssignal s(t) gemäss Fig. 2 wird durch die Transducerelemente 1 in die jeweiligen akustischen Sende-Wellenpakete s'(t) gewandelt, welche ins Schallmedium propagieren, wo Anteile davon, durch die im Schallmedium stationär enthaltene oder, in einem Fluidum als Schallmedium, darin bewegte reflektierende Struktur reflektiert werden.

Gemäss Fig. 1 wird nun die Schichtfläche 4, welche auszumessen ist, in eine Anzahl M Volumenelemente unterteilt, wie dargestellt bevorzugterweise gleich grosse, wobei in Fig. 1 der Spezialfall dargestellt ist, in dem die Schicht 4 in einer parallel zur Transducerebene liegenden Ebene liegt.

Es werde nun den gesamthaft M vorgesehenen Volumenelementen die Laufnummer m mit 1 < m < M zugeordnet und es sei weiter die Transducermatrix 1a mit gesamthaft N Transducerelementen 1 versehen, welche mit der Laufnummer n mit 1 < n < N numeriert seien. Es erzeugt beim Transducerelement No. n das Volumenelement No. m folgendes Echosignal:

$$e_{nm}(t) = D A(t - \tau - r_{nm}/c)e^{j\omega_o(t - \tau) - jkr_{nm}} \quad (3)$$

mit k = $\omega_0$/c : Wellenzahl c: Schallgeschwindigkeit D: Verlustfaktor

Dabei bedeutet $\tau$ die Laufzeit des Sende-Wellenpaketes von der Transducerebene zur Schicht 4, wobei diese Laufzeit im betrachteten Spezialfall für alle Volumenelemente m gleich gesetzt wird. D berücksichtigt die Dämpfung des Schalls im Medium sowie Streuverluste.

Die Phase des Empfangssignals an einem Transducerelement No. n von einem Volumenelement No. m, d.h. das Echosignal $e_{nm}$ reagiert sehr empfindlich auf Aenderungen der Distanz $r_{nm}$, welche von einem betrachteten Transducerelement 1 zu einem betrachteten Volumenelement definiert ist. (Im allgemeinen Fall sind analog unterschiedliche $\tau_m$ zu berücksichtigen.) Von einem betrachteten Volumenelement No. m empfängt mithin jedes Transducerelement 1 der Matrix 1a ein Echosignal in einer anderen Phasenlage, bedingt durch die unterschiedlichen Distanzen der Volumenelemente 4a zum einen betrachteten Transducerelement 1. Die Amplitude A(t) hingegen ist eine verhältnismässig mit der Distanz wenig variierende Grösse.

Wie in Fig. 2 dargestellt, erscheint am elektrischen Ausgang jedes Transducerelementes 1, beispielsweise desjenigen mit der No. 1, ein Echo, welches sich aus der Gesamtheit des von jedem Volumenelement erzeugten Echos $e_{1m}$ ergibt. Dieses Echosignal jedes Transducerelementes 1 wird am Empfänger 7 verstärkt, und darnach am Synchrondemodulator 8 demoduliert.

In der bevorzugten dargestellten Ausführungsvariante werden nun, wie auch in Fig. 4e dargestellt, zu wenigen vorgegebenen Abtastzeitpunkten $t_i$ die orthogonalen Signale ausgangsseitig des Synchrondemodulators 8 abgetastet, wobei (hier nicht dargestellt) in üblicher Art und Weise vom demodulierten Signal erst Signalanteile mit der Summenfrequenz 2 $\omega_o$ ausgefiltert werden. Ausgangsseitig des Synchrondemodulators erscheint, bezogen auf das beispielsweise besprochene Transducerelement No. 1, die Summe aller komplexen Volumenelement-Echo-Enveloppen, wobei sich, von einem Volumenelement m, diese komplexe Enveloppe, bezogen auf ein Transducerelement n, ergibt gemäss:

$$c_{nm}(t) = D A(t - \tau - r_{nm}/c)e^{- j\omega_o\tau - jkr_{nm}} \quad (4)$$

Dieses Signal enthält die volle Information des HF-Signals. Da das Signal bandbegrenzt ist, genügen zur Digitalisierung einige wenige, z.B. vier aequidistant liegende Stützpunkte bei den Abtastzeiten $t_i$, wie in Fig. 4e dargestellt. Die nun zu den erwähnten Zeitpunkten $t_i$ abgetasteten demodulierten Signale werden, wie erwähnt wurde, an den A/D-Wandlern 10 gewandelt und in digitalisierter Form der Multiplexereinheit 11 zugeführt. Ueber die Multiplexereinheit 11 werden sie nun in die Speichereinheit 12 geladen. Im Speicher 12 liegen mithin die digitalisierten Summen der komplexen Enveloppen-Signale, zugeordnet den Transducerelement-No. n=1, 2 ... N.

Es geht nun darum, aus diesem Summensignal - abgetastete (∗) Summen der komplexen Enveloppen $c_{nm}$ - zu strukturieren, welcher Echoanteil von welchem Volumenelement an einem betrachteten Transducerelement herrührt.

Nun sind in einem Speicher 12a, beispielsweise einem ROM-, PROM-, EPROM-Speicher, zu jedem Transducerelement n=1 bis N, die Distanzgrössen $r_{nm}$ zu den jeweiligen Volumenelementen 4a in der Schicht 4 gemäss Fig. 1 abgespeichert.

Eigentlich wird durch Festlegung dieser r-Distanzgrössen festgelegt, wie die Schicht 4 gemäss Fig. 1 bezüglich der Transduceranordnung 1a liegen soll.

Aus diesen unterschiedlichen Distanzgrössen $r_{nm}$, womit eigentlich Volumenelement/Transducerelement-Laufzeit-spezifische Phasenunterschiede $\phi_{nm}$ definiert sind, werden "virtuelle" SOLL-Abtastzeiten $t_{nma}$ berechnet, bei denen das jeweilige Transducerelementspezifische Summenechosignal am Ausgang des Synchrondemodulators 8 abgetastet werden müsste, damit, zu diesen vorgegebenen Zeitpunkten abgetastet, die so abgetasteten Signale dem Signalanteil entsprächen, der je von einem betrachteten Volumenelement auf das betrachtete Transducerelement 1 auftrifft.

Nun werden einerseits vom Speicher 12 die jedem Transducerelement zugeordneten Summensignale der komplexen Enveloppen einem Interpolator 13 zugeführt, ebenso wie die ausgehend von den Distanzwerten $r_{nm}$ ermittelten SOLL-Abtastzeiten $t_{nma}$. Im Interpolator werden nun die eigentlich benötigten Werte der komplexen Enveloppensumme durch numerische Interpolation zu den eigentlich erforderlichen Volumenelement-Transducerelement-Abtastzeiten $t_{nma}$ ermittelt.

Es resultieren ausgangsseitig des Interpolators die an jedem Transducerelement der No. n ermittelten, komplexen Enveloppen jedes Volumenelementes m, unter Berücksichtigung der Phasendrehung entsprechend den fiktiven Abtastzeiten bzw. Interpolations-Abtastzeiten $t_{nma}$. Die nun zu den Volumenelement/Sensor-spezifischen Abtastzeiten $t_{nma}$ rechnerisch durch Interpolation ermittelten Werte sind aber weiterhin in dem Sinne "verrauscht", als dass auch bei diesen $t_{nma}$-Abtastwerten noch von anderen Volumenelementen 4a herrührende Anteile vorhanden sind. Die gemäss den $t_{nma}$-Tastzeiten interpolierten Signale werden nun in den Speicher 14 geladen, worin, nun matrixartig, jedem Transducerelement, für jedes Volumenelement die so interpolierte komplexe Enveloppen-Summe aus den Enveloppen:

$$c_{nm} * \; = \; c_{nm}(t_i)e^{\,j\omega_0(t_{nma} - \, t_i)} \; = \; c_{nm}(t_i)e^{j\phi_{nm}}, \; i \; = \; 1,2,3 \text{ oder } 4 \quad (5)$$

zugeordnet sind.

Dabei bezeichnet:

$\phi_{nm}$ : eine von der Distanz $r_{nm}$ abhängige Phasendifferenz.

Der Interpolator 13 führt diese komplexe Multiplikation durch, wobei er aus dem Tabellenspeicher 12a die den jeweiligen Volumenelementen bzw. Transducerelementen zugehörigen Werte ausliest.

Die Summen der komplexen Enveloppen im Speicher 14 bestehen je aus Abtastwerten der an jedem Transducerelement empfangenen Gesamtechos, die dann aufgenommen sind, wenn an jedem Transducerelement das Echo von je einem Volumenelement aufgrund der Transducerelement-Volumenelement-Distanz, eintrifft.

Betrachtet man diese Abtastwerte, die wie erwähnt, noch verrauscht sind, für ein Volumenelement an allen Transducerelementen, so entsprechen sie, da die Distanzunterschiede durch die spezifischen $t_{nma}$-Interpolationszeiten berücksichtigt sind, kohärent an allen Transducerelementen ankommenden Echosignalen von jeweils einem Volumenelement, verrauscht durch nichtkohärent angekommene Echosignale der anderen Volumenelemente, für die die jeweiligen $t_{nma}$-Zeitpunkte nicht stimmen. Im Sinne dieser Kohärenz sind somit an allen Transducerelementen die Echos von einem Volumenelement nun korreliert.

Es werden nun alle von einem Volumenelement abgespeicherten komplexen Summen Enveloppenwerte aus den $\Sigma * c_{nm}$, über alle Transducerelemente, wie mit der Umschaltereinheit 14a schematisch angedeutet, an einem Summator 15 aufsummiert, wobei durch geeignete Wahl der Gewichtungsfaktoren $W_n$ das Uebersprechen zwischen den einzelnen Volumenelementen noch weiter unterdrückt werden kann. Die erwähnte Korreliertheit tritt nun gegenüber der Nicht-Korreliertheit verstärkt hervor. Am Ausgang des Summators 15 treten somit der Reihe nach Summensignale $C_m$ auf, d.h. die über alle Transducerelemente 1 summierten Summen-Echosignale von einem jeweils betrachteten Volumenelement mit der No. m. Diese Summensignale (von Summensignalen mit korreliertem "Summanden") ergeben sich nach:

$$C_m = \sum_{n=1}^{N} N_n \cdot \sum_{1}^{M} c_{nm}^* (t_i - t_{nma})$$ (6)

Diese nur mehr Volumenelement-spezifischen Echosignale werden in einem Speicher 16 abgelegt.

Die komplexen Werte $C_m$ enthalten Information über die im Volumenelement enthaltenen reflektierenden Strukturen: Der Betrag ist ein Mass für die Reflexion, die Phase hängt von der relativen Position der reflektierenden Strukturen ab. Somit bildet der Ausgang bzw. Inhalt des Speichers 16 bei der Anwendung des erfindungsgemässen Verfahrens bzw. der erfindungsgemässen Vorrichtung für Echografie, d.h. für nichtbewegte Strukturen, bereits das Resultat.

Die bis anhin beschriebene Technik ist aber insbesondere geeignet für die Messung der Gechwindigkeitsverteilung der Struktur. Hierzu werden, wie bereits in Fig. 4a dargestellt, mindestens zwei Sende-Wellenpakete s'(t) abgesandt und die Echos beider, durch die lange Zeit T getrennten Pakete, je, wie bis anhin erläutert wurde, ausgewertet.

Es stehen somit durch die mindestens zwei Sende-Wellenpakete und ihre Auswertung im Speicher 16, von jedem Volumenelement mindestens zwei komplexe Werte $C_m$ und $C'_m$ zur Verfügung. Aus Klarheitsgründen und um nicht die ganze Auswertungsanordnung doppelt erläutern zu müssen, sind lediglich im Endspeicher 16 die bei zwei oder mehr Wellenpakten resultierenden, je den Volumenelementen zugeordneten komplexen Werte $C_m$ und $C'_m$ eingetragen.

Aus der Phasenänderung $\Delta \phi_m(C, C')$, d.h. der Phasenänderung der komplexen Werte $C_m$ von Wellenpaketecho zu Wellenpaketecho wird die Geschwindigkeit $v_m$ der Struktur im Sinne der Doppler-Frequenzauswertung bestimmt, d.h. in Beschallungsrichtung.

Dazu werden, im bevorzugten Fall, bei mehreren Sende-Wellenpaketen, eine zeitliche Folge von Werten $C_m$, $C'_m$, $C''_m$ etc., welche Stützwerte der in den m-ten Volumenelementen generierten Doppler-Frequenzen darstellen, in bekannter Art und Weise analysiert. Ein digitales Hochpassfilter (nicht dargestellt) unterdrückt die stationären Anteile, die z.B. von Gefässwänden herrühren können und im allgemeinen dem dopplerverschobenen Signal überlagert sind. Das gefilterte Signal gelangt sodann zum Frequenzdetektor 17, welcher die mittlere Dopplerfrequenz bestimmt und damit pro Volumenelement die Geschwindigkeit $v_m$ der Struktur.

Durch Summation der für jedes Volumenelement m so ermittelten Doppler-Frequenz $f_{Dm}$ kann der Volumenstrom $\dot{Q}$ durch die Schicht 4 gemäss Fig. 1 nach folgender Formel bestimmt werden:

$$\dot{Q} = \frac{c}{2f_0} \sum_{1}^{M} F_m f_{Dm}$$ (7)

$F_m$ :     Fläche des auf die Messebene projizierten Volumenelementes, Messebene senkrecht zur Beschallungsrichtung

c :     Schallgeschwindigkeit

$f_0$ :     Frequenz von r(t)

$f_{Dm}$:     Dopplerfrequenz im m-ten Volumenelement

Der Gewichtungsfaktor $F_m$ berücksichtigt dabei die Grösse der Teilflächen der Volumenelemente m.

Der Volumenstrom $\dot{Q}$, der so bezüglich einer zur Transducermatrixfläche parallelen Schicht ermittelt wird, ist unabhängig von der Richtung der Geschwindigkeitsvektoren durch diese Schicht.

## Patentansprüche

1.     Verfahren zum Messen einer zweidimensionalen reflektierenden Struktur in einer Schicht (4) eines Materialvolumens mittels Auswertung von Schall- oder Ultraschallecho ($e_{nm}$), bei dem
- mindestens ein Sende-Wellenpaket (s'(t)) erzeugt wird,
- eine Mehrzahl Schall- oder Ultraschallsensoren (1) in einer mindestens eindimensionalen Anordnung(1a) vorgesehen wird,
- die Echo-Empfangssignale jeweils einer Anzahl Sensoren auf ein Volumenelement der Schicht durch Berücksichtigung von Laufzeitunterschieden zwischen Volumenelementen und den Sensoren der Anzahl elektronisch fokussiert werden,
dadurch gekennzeichnet, dass

- gleichermassen aus dem Echo-Empfangssignal jedes Sensors (1....N), allen Volumenelementen (1....M) der Schicht zugeordnete Volumenelement-Echosignalwerte ($\Sigma * c_{nm}$) zu die Laufzeitunterschiede berücksichtigenden Zeiten ($t_i$, $t_{nma}$) durch Abtasten und Interpolation ermittelt werden
- die je einem Volumenelement (1...M) zugeordneten Volumenelement-Echosignal-Werte ($\Sigma * c_{nm}$) über alle Sensoren (1....N) kombiniert werden ($\Sigma W_{12} \Sigma * c_{nm}$) zum Erhalt eines Gesamt-Volumenelement-Echosignal-Wertes ($C_m$), das representativ ist für die reflektierende Struktur im betrachteten Volumenelement.

2. Verfahren nach Anspruch 1 zum Messen des zweidimensionalen Geschwindigkeitsfeldes der reflektierenden Struktur in der Schicht (4), dadurch gekennzeichnet, dass mindestens zwei der Sende-Wellenpakete ($s'(t)$) zeitgestaffelt (T) erzeugt werden, ihre Echos ausgewertet werden ($C_m$, $C'_m$) dabei durch zusätzliches Auswerten der Phase der Gesamt-Volumenelement-Echosignal-Werte ($\Delta \phi(C_m, C'_m)$) ein zur Geschwindigkeit der Struktur in den Volumenelementen (4a) proportionales Volumenelement-Geschwindigkeitssignal ($v_m$) gebildet wird.

3. Verfahren nach einem der Ansprüche 1 oder 2, dadurch gekennzeichnet, dass das Sende-Wellenpaket ($s'(t)$) so erzeugt wird, dass es die Schicht (4) mindestens genähert kohärent, d.h. mindestens genähert phasengleich, erreicht.

4. Verfahren nach einem der Ansprüche 1 bis 3, dadurch gekennzeichnet, dass das Sende-Wellenpaket ($s'(t)$) durch zeit- und phasenrichtige Anregung ($s(t)$) einer Mehrzahl von Schall- oder Ultraschallquellen (1) erzeugt wird.

5. Verfahren nach einem der Ansprüche 1 bis 4, dadurch gekennzeichnet, dass das Sende-Wellenpaket ($s'(t)$) durch mindestens genähert gleichzeitige Anregung einer Mehrzahl mindestens genähert in einer Ebene (1a) angeordneter Schall- oder Ultraschallquellen (1) erzeugt wird.

6. Verfahren nach einem der Ansprüche 1 bis 5, dadurch gekennzeichnet, dass die Sensoren (1) matrixförmig (1a), mindestens genähert in einer Ebene angeordnet werden.

7. Verfahren nach einem der Ansprüche 1 bis 6, dadurch gekennzeichnet, dass Quellen (1) zur Erzeugung des Sende-Wellenpakets ($s'(t)$) und die Sensoren (1) mittels einer gemeinsamen Anordnung von Sende- und Empfangstransducern (1) gebildet werden, vorzugsweise einer mindestens genähert ebenen (1a).

8. Verfahren nach einem der Ansprüche 1 bis 7, dadurch gekennzeichnet, dass das Sende-Wellenpaket ($s'(t)$) mittels einer Mehrzahl Schall- oder Ultraschallquellen (1) erzeugt wird, die je von einem, von einem stabilen, vorzugsweise quarzstabilen Referenzsignal ($r(t)$) mindestens abgeleiteten Anregungssignal angeregt werden.

9. Verfahren nach Anspruch 8, dadurch gekennzeichnet, dass die Echoempfangssignale mit dem Referenzsignal ($r(t)$) demoduliert werden.

10. Verfahren nach einem der Ansprüche 1 bis 9, dadurch gekennzeichnet, dass die Echo-Empfangssignale digitalisiert (10) werden, vorzugsweise nach deren Demodulation (8).

11. Verfahren nach einem der Ansprüche 1 bis 10, dadurch gekennzeichnet, dass die Echo-Empfangssignale von jedem Sensor (1) digitalisiert werden, die unterschiedlichen Laufzeiten, entsprechend unterschiedlichen Echos-Empfangszeiten von Volumenelementechos, an allen Senoren (1) durch das Abtasten und Interpolieren ($t_{nma}$) phasenkompensiert werden, derart, dass von allen Sensoren (1) gleichphasige, laufzeitkompensierte Volumenelement-Echosignale ($\Sigma * c_{nm}$) vorliegen.

12. Verfahren nach einem der Ansprüche 1 bis 11, dadurch gekennzeichnet, dass die Kombination der Volumenelement-Echosignalwerte so erfolgt, dass dabei die Signalwerte jeweils von einem Volumenelement (m) verstärkt werden, gegenüber Signalwerten von allen anderen Volumenelementen.

13. Verfahren nach einem der Ansprüche 2 bis 12, dadurch gekennzeichnet, dass durch, gegebenenfalls gewichtete, Summation der Volumenelement-Geschwindigkeitssignale ($v_m$) der Volumenstrom ($\dot{Q}$) durch die Schicht bestimmt wird.

14. Messanordnung zum Messen einer zweidimensionalen reflektierenden Struktur in einer Schicht (4), durch ein Materialvolumen, mittels Auswertung von Schall- oder Ultraschallechos mit
    - einer Sende-Anordnung (1,1a) zur Beschallung des Materialvolumens mit mindestens einem Sende-Wellenpaket
    - einer mindestens eindimensionalen Anordnung einer Mehrzahl von Schall- oder Ultraschallsensoren, wovon jeweils eine Anzahl durch Mittel, mittels welchen Laufzeitunterschiede zwischen Sensoren und Volumenelementen des Materialvolumens berücksichtigt werden, elektronisch auf ein Volumenelement (4a) fokussiert sind,
    dadurch gekennzeichnet, dass
    - der Ausgang jedes Sensors gleichwertig einer Abtasteinheit (9), ihr nachgeschaltet einer Interpolationseinheit (13), ihr nachgeschaltet einer Speichereinheit (14) zugeführt ist, woran, gesteuert durch Laufzeitwerte in einem Laufzeitspeicher (12a), Signalwerte abgetastet, interpoliert und abgespeichert werden, die den Echosignalen jedes Volumenelementes am betrachteten Sensor entsprechend
    - die abgetasteten Signalwerte, die dem Echosignal eines Volumenelementes entsprechen und vom Ausgang jedes Sensors gebildet sind, ausgangsseitig der Speichereinheit (14) einer Korrelationseinheit (15) zugeführt sind
    - ausgangsseitig der Korrelationseinheit (15) ein Volumenelement-Echosignalspeicher (16) vorgesehen ist, woran pro Volumenelement ein Echosignalwert abgespeichert wird.

15. Messanordnung nach Anspruch 14, dadurch gekennzeichnet, dass die Korrelationseinheit (15) einen Summator (15) umfasst.

16. Messanordnung nach einem der Ansprüche 14 oder 15, dadurch gekennzeichnet, dass der Senderanordnung (1) eine Anregungseinheit (6) zugeschaltet ist, die sie zur Abgabe mindestens von zwei zeitlich getrennten Sende-Wellenpaketen (s'(t)) anregt.

17. Messanordnung nach Anspruch 16, dadurch gekennzeichnet, dass im Volumenelement-Echospeicher (16) für die Sende-Wellenpakete die Echosignalwerte abgespeichert sind und dass ihm ein Frequenz- oder Phasendetektor (17) nachgeschaltet ist, um aus den gespeicherten Ausgangssignalwerten der Korrelationseinheit (15), auf Basis des Dopplereffektes, die Strukturgeschwindigkeit in den Volumenelementen der Schicht (4) zu berechnen.

18. Messanordnung nach Anspruch 17, dadurch gekennzeichnet, dass dem Phasen- bzw. Frequenzdetektor (17) ein Summator (18) nachgeschaltet ist, um aus den Geschwindigkeitssignalen den Volumenfluss durch die Schicht zu berechnen.

## Claims

1. Process for measuring a two-dimensional reflecting structure in a layer (4) of a material volume by means of evaluation of the sound or ultrasound echo ($e_{nm}$), where
   - at least one transmitting wave bundle (s'(t)) is generated,
   - a multiplicity of sound or ultrasound sensors (1) is provided in an at least one-dimensional arrangement (1a),
   - the echo receiving signals of a number of sensors in each case are electronically focused on one volume element in the layer by taking account of the differences in transit time between volume elements and the sensors of the said number,
   characterised in that
   - volume element echo signal values ($\Sigma * C_{nm}$) associated with all volume elements (1....M) in the layer are determined in a like manner from the echo receiving signal of each sensor (1....N) by scanning and interpolation at times ($t_i$, $t_{nma}$) which take account of the differences in transit time,
   - the volume element echo signal values ($\Sigma * C_{nm}$) each assigned to one volume element (1....M) are combined ($\Sigma W_{12} \Sigma * C_{nm}$) via all sensors (1....N) to give a total volume element echo signal value ($C_m$) which is representative of the reflecting structure in the volume element concerned.

2. Process according to Claim 1 for measuring the two-dimensional velocity field of the reflecting structure in the layer (4), characterised in that at least two of the transmitting wave bundles (s'(t)) are generated at different times (T), their echos are evaluated ($C_m$, $C'_m$) and a volume element velocity signal ($v_m$) proportional to the velocity of the structure in the volume elements (4a) is formed by additional evaluation of the phase of the total volume element echo signal values ($\Delta \phi$ ($C_m$, $C'_m$)).

3. Process according to one of Claims 1 or 2, characterised in that the transmitting wave bundle (s'(t)) is generated such that it reaches the layer (4) at least approximately coherently, i.e. at least approximately in phase.

4. Process according to any of Claims 1 to 3, characterised in that the transmitting wave bundle (s'(t)) is generated by the excitation (s(t)) at the correct time and phase of a multiplicity of sound or ultrasound sources (1).

5. Process according to any of Claims 1 to 4, characterised in that the transmitting wave bundle (s'(t)) is generated by the at least approximately simultaneous excitation of a multiplicity of sound or ultrasound sources (1) arranged at least approximately in one plane (1a).

6. Process according to any of Claims 1 to 5, characterised in that the sensors (1) are arranged in a matrix (1a) at least approximately in one plane.

7. Process according to any of Claims 1 to 6, characterised in that sources (1) for generating the transmitting wave bundle (s'(t)) and the sensors (1) are formed by means of a common arrangement of transmitting and receiving transducers (1), preferably in an at least approximately flat arrangement (1a).

8. Process according to any of Claims 1 to 7, characterised in that the transmitting wave bundle (s'(t)) is generated by means of a multiplicity of sound or ultrasound sources (1) which are each excited by an excitation signal which is derived at least from a stable and preferably quartz-stable reference signal (r(t)).

9. Process according to Claim 8, characterised in that the echo receiving signals are demodulated with the reference signal (r(t)).

10. Process according to any of Claims 1 to 9, characterised in that the echo receiving signals are digitalised (10), preferably after their demodulation (8).

11. Process according to any of Claims 1 to 10, characterised in that the echo receiving signals from each sensor (1) are digitalised, the different transit times are phase-compensated at all the sensors (1) according to different echo receiving times of volume element echos by scanning and interpolation ($t_{nma}$) so as to give identical-phase, transit time-compensated volume element echo signals ($\Sigma * C_{nm}$) from all sensors (1).

12. Process according to any of Claims 1 to 11, characterised in that the volume element echo signal values are combined in such a way that the signal values from each volume element (m) are amplified in comparison with signal values from all the other volume elements.

13. Process according to any of Claims 2 to 12, characterised in that the volume flow (Q) through the layer is determined by summation, where applicable weighted, of the volume element velocity signals ($v_m$).

14. Measuring arrangement for measuring a two-dimensional reflecting structure in a layer (4) through a material volume by the evaluation of sound or ultrasound echos with
   - a transmitting arrangement (1,1a) for the acoustic irradiation of the material volume with at least one transmitting wave bundle
   - an at least one-dimensional arrangement of a multiplicity of sound or ultrasound sensors, a number of which in each case are electronically focused on one volume element (4a) through means which take account of transit time differences between sensors and volume elements of the material volume,

   characterised in that
   - the output of each sensor is fed equally to a scanning unit (9), an interpolation unit (13) connected after this, and a storage unit (14) connected after this where, controlled by transit time values in a transit time storage unit (12a), signal values which correspond to the echo signals of each volume element at the sensor concerned are scanned, interpolated and stored,
   - the scanned signal values which correspond to the echo signal of one volume element and are formed by the output of each sensor are fed to a correlation unit (15) on the output side of the storage unit (14),

- on the output side of the correlation unit (15) is a volume element echo signal storage unit (16), at which is stored one echo signal value per volume element.

**15.** Measuring arrangement according to Claim 14, characterised in that the correlation unit (15) comprises a summator (15).

**16.** Measuring arrangement according to one of Claims 14 or 15, characterised in that the transmitter arrangement (1) is connected to an excitation unit (6) which excites it for the purpose of emitting at least two transmitting wave bundles (s'(t)) at different times.

**17.** Measuring arrangement according to Claim 16, characterised in that the echo signal values are stored in the volume element echo storage unit (16) for the transmitting wave bundles, and that a frequency or phase detector (17) is connected after this in order to calculate, using the Doppler effect, the structural velocity in the volume elements of the layer (4) from the stored output signal values of the correlation unit (15).

**18.** Measuring arrangement according to Claim 17, characterised in that a summator (18) is connected after the phase or frequency detector (17) in order to calculate from the velocity signals the volume flow through the layer.

**Revendications**

**1.** Procédé pour mesurer une structure réfléchissante à deux dimensions dans une couche (4) d'un volume de matière à l'aide de l'évaluation de l'écho de sons ou d'ultrasons ($e_{nm}$), selon lequel
   - au moins un paquet d'ondes d'émission (s'(t)) est généré,
   - une multiplicité de capteurs de sons et d'ultrasons (1) est prévue dans un dispositif à au moins une dimension (1a),
   - les signaux de réception d'écho d'un certain nombre de capteurs sont concentrés par voie électronique sur un élément de volume de la couche, en tenant compte de différences de durée de propagation entre les éléments de volume et lesdits capteurs,
   caractérisé en ce que
   - à partir du signal de réception d'écho de chaque capteur (1....N), les valeurs de signaux d'écho d'éléments de volume ($\Sigma * c_{nm}$) associées à tous les éléments de volume (1....M) de la couche sont calculées de la même manière par balayage et interpolation à des moments ($t_1$, $t_{nma}$) qui tiennent compte des différences de durée de propagation, et
   - les valeurs de signaux d'écho d'éléments de volume ($\Sigma * c_{nm}$) associées à un élément de volume (1....M) sont combinées ($\Sigma W_{12} \Sigma * c_{nm}$) par l'intermédiaire de tous les capteurs (1....N) en vue d'obtenir une valeur globale de signal d'écho d'élément de volume ($C_m$) représentative de la structure réfléchissante dans l'élément de volume considéré.

**2.** Procédé selon la revendication 1 pour mesurer le champ de vitesse à deux dimensions de la structure réfléchissante dans la couche (4), caractérisé en ce qu'au moins deux des paquets d'ondes d'émission (s'(t)) sont générés de façon échelonnée dans le temps (T) et leurs échos sont évalués ($C_m$, $C'_m$), un signal de vitesse d'élément de volume ($v_m$) proportionnel à la vitesse de la structure dans les éléments de volume (4a) étant formé grâce à l'évaluation supplémentaire de la phase des valeurs globales de signaux d'échos d'éléments de volume ($\Delta \phi(C_m, C'_m)$).

**3.** Procédé selon l'une des revendications 1 ou 2, caractérisé en ce que le paquet d'ondes d'émission (s'(t)) est généré de telle sorte qu'il atteint la couche (4) de façon au moins approximativement cohérente, c'est-à-dire au moins approximativement en phase.

**4.** Procédé selon l'une des revendications 1 à 3, caractérisé en ce que le paquet d'ondes d'émission (s'(t)) est généré grâce à l'excitation (s(t)) suivant la bonne durée et la bonne phase d'une multiplicité de sources de sons ou d'ultrasons (1).

**5.** Procédé selon l'une des revendications 1 à 4, caractérisé en ce que le paquet d'ondes d'émission (s'(t)) est généré grâce à l'excitation au moins approximativement simultanée d'une multiplicité de sources de sons ou d'ultrasons (1) disposées au moins approximativement dans un plan (1a).

6. Procédé selon l'une des revendications 1 à 5, caractérisé en ce que les capteurs (1) sont disposés sous forme de matrice (1) au moins approximativement dans un plan.

7. Procédé selon l'une des revendications 1 à 6, caractérisé en ce que des sources (1) destinées à générer le paquet d'ondes d'émission (s'(t)) et les capteurs (1) sont définis à l'aide d'un agencement commun de transducteurs d'émission et de réception (1), de préférence au moins approximativement plan (1a).

8. Procédé selon l'une des revendications 1 à 7, caractérisé en ce que le paquet d'ondes d'émission (s'(t)) est généré à l'aide d'une multiplicité de sources de sons ou d'ultrasons (1) qui sont excitées chacune par un signal d'excitation au moins dérivé d'un signal de référence (r(t)) stable, de préférence stabilisé par cristal.

9. Procédé selon la revendication 8, caractérisé en ce que les signaux de réception d'écho sont démodulés à l'aide du signal de référence (r(t)).

10. Procédé selon l'une des revendications 1 à 9, caractérisé en ce que les signaux de réception d'écho sont numérisés (10), de préférence après leur démodulation (8).

11. Procédé selon l'une des revendications 1 à 10, caractérisé en ce que les signaux de réception d'écho sont numérisés par chaque capteur (1) et les différentes durées de propagation sont soumises au niveau de tous les capteurs (1), grâce au balayage et à l'interpolation ($t_{nma}$), à une compensation de phase en fonction des différentes durées de réception d'échos d'éléments de volume, de telle sorte qu'on a au niveau de tous les capteurs (1) des signaux d'écho d'éléments de volume ($\Sigma * C_{nm}$) en phase à durée de propagation compensée (1).

12. Procédé selon l'une des revendications 1 à 11, caractérisé en ce que la combinaison des valeurs de signaux d'écho des éléments de volume se fait de telle sorte que les valeurs de signaux sont amplifiées à chaque fois par un élément de volume (m) par rapport aux valeurs de signaux de tous les autres éléments de volume.

13. Procédé selon l'une des revendications 2 à 12, caractérisé en ce que le flux de volume ($\dot{Q}$) à travers la couche est déterminé par la somme, éventuellement pondérée, des signaux de vitesse d'éléments de volume ($v_m$).

14. Dispositif de mesure pour mesurer une structure réfléchissante à deux dimensions dans une couche (4), à travers un volume de matière, à l'aide de l'évaluation d'échos de sons ou d'ultrasons, comportant
    - un dispositif émetteur (1, 1a) pour exposer le volume de matière à l'action des ultrasons à l'aide d'au moins un paquet d'ondes d'émission,
    - un agencement à au moins une dimension d'une multiplicité de capteurs de sons ou d'ultrasons dont un certain nombre est concentré par voie électronique sur un élément de volume (4a) grâce à des moyens à l'aide desquels les différences de durée de propagation entre les capteurs et les éléments du volume de matière sont prises en compte, caractérisé en ce que
    - la sortie de chaque capteur est reliée de façon équivalente à une unité de balayage (9), à une unité d'interpolation (13) montée en aval et à une unité de mémoire (14) montée en aval de celle-ci, des valeurs de signaux étant balayées, interpolées et mémorisées, commandées par des valeurs de durée de propagation dans une mémoire de durées de propagation (12a), lesquelles valeurs de signaux correspondent aux signaux d'écho de chaque élément de volume au niveau du capteur considéré,
    - les valeurs de signaux balayées qui correspondent au signal d'écho d'un élément de volume et qui sont définies par la sortie de chaque capteur sont transmises, du côté de la sortie de l'unité de mémoire (14), à une unité de corrélation (15),
    - du côté de la sortie de l'unité de corrélation (15), il est prévu une mémoire de signaux d'écho d'éléments de volume (16) au niveau de laquelle une valeur de signal d'écho est mémorisée pour chaque élément de volume.

15. Dispositif de mesure selon la revendication 14, caractérisé en ce que l'unité de corrélation (15) comprend un sommateur (15).

**16.** Dispositif de mesure selon l'une des revendications 14 ou 15, caractérisé en ce qu'il est prévu, raccordée au dispositif émetteur (1), une unité d'excitation (6) qui l'excite en vue de la sortie d'au moins deux paquets d'ondes d'émission (s'(t)) séparés dans le temps.

**17.** Dispositif de mesure selon la revendication 16, caractérisé en ce que les valeurs de signaux d'écho sont mémorisées dans la mémoire d'échos d'éléments de volume (16) pour les paquets d'ondes d'émission, et en ce qu'un détecteur de fréquence ou de phase (17) est monté en aval de ladite mémoire afin de calculer, à partir des valeurs de signaux de sortie de l'unité de corrélation (15) mémorisées, et sur la base de l'effet Doppler, la vitesse de structure dans les éléments de volume de la couche (4).

**18.** Dispositif de mesure selon la revendication 17, caractérisé en ce qu'un sommateur (18) est monté en aval du détecteur de phase ou de fréquence (17), afin de calculer le flux de volume à travers la couche à partir des signaux de vitesse.

F I G. 1

FIG. 2

FIG. 3

FIG. 4

a) A(t)

T

b) r(t)

c) s(t)

d) $e_{nm}(t)$

$\tau + \dfrac{r_{nm}}{c}$

e) Abtast-Zeitpunkte $t_i$

$c_{nm}(t)$

$t_1$ $t_2$ $t_3$ $t_4$

EP 0 394 428 B1